# EUROPEAN PATENT APPLICATION

(11) **EP 3 150 251 A1**
(43) Date of publication of application: **05.04.2017**
(21) Application number: 15002809.0
(22) Date of filing: 01.10.2015
(51) Int. Cl.: A61N 1/36, A61B 5/0402

(54) **ELECTROTHERAPEUTIC APPARATUS AND METHOD OF CONTROLLING THE SAME**

(71) Applicant: Chao, Hsing-Rong, Taoyuan City 330 (TW)
(72) Inventor: Chao, Hsing-Rong, Taoyuan City 330 (TW)
(74) Representative: Zeitler Volpert Kandlbinder Patent- und Rechtsanwälte Partnerschaft mbB

(57) **Abstract**

The invention provides improved electrotherapeutic apparatus which detect a vibrational frequency of heart blood vessel and then produce a stimulating frequency current corresponding to the vibrational frequency of heart blood vessel. The improved electrotherapeutic apparatus comprises: a detecting module which detects a vibrational frequency of heart blood vessel and produces a signal of the vibrational frequency of heart blood vessel, a processing module which receives the signal of vibrational frequency of heart blood vessel and produces a response signal and a action module which receives the response signal and then produces a stimulating current corresponding to the vibrational frequency of heart blood vessel and then adjusts stimulating signal frequency to enhance the therapeutic effect of pain and rehabilitation.

## Description

### BACKGROUND OF THE INVENTION

### 1. Field of the Invention

The invention relates to an electrotherapeutic apparatus. In more detail, the invention relates to an improved electrotherapeutic apparatus which detects a vibrational frequency of heart blood vessel and then produces a stimulating current corresponding to the vibrational frequency of heart blood vessel and the method of controlling the same.

### 2. Description of the Prior Art

According to the theory of Chinese acupuncture and moxibustion therapy, it has therapeutic effect of pain and rehabilitation. However, when Chinese acupuncture and moxibustion are operated, the needles have to invade into the body. Hence, Chinese acupuncture and moxibustion usually cause rejection reaction due to different personal physique and the needles. Therefore, currently Chinese acupuncture and moxibustion apply current stimulation to replace traditional therapeutic methods.

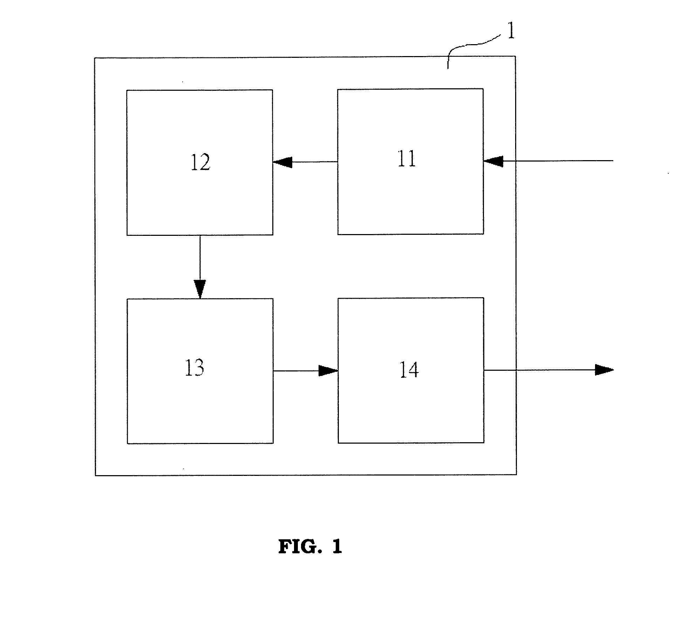

Generally, the electrotherapeutic apparatus sold in the current market is operated at constant frequency to release pain or do rehabilitation therapy. However, when the physical situation is different, the frequency is also required to change. As a result, the electrotherapeutic apparatus with a constant frequency does not give a good therapeutic effect related to release pain and do rehabilitation therapy. Referring to FIG 1, FIG 1 is a drawing of the therapeutic apparatus 1 disclosed in TW M428769. The therapeutic apparatus 1 disclosed in TW M428769 comprises: an oscillator 11, a variable resistor 12, a transistor13 and a coil transformer 14. The oscillator 11 is used to adjust the frequency produced by electrotherapeutic apparatus 1 and then uses the transistor 13 to enlarge the current. The coil transformer 14 is used to enlarge the voltage so as to produce different stimulating signal frequency. However, the apparatus and method disclosed in M428769 is conduct by personal feeling to adjust the stimulating signal frequency, so the stimulating signal frequency is not well controlled. When the stimulating signal frequency is too high, it will damage the body. In contrast, when the stimulating signal frequency is too low, there is no therapeutic effect. Therefore, there is a need to develop an improved electrotherapeutic apparatus with a variable frequency.

### SUMMARY OF THE INVENTION

According to the disadvantage of electrotherapeutic apparatus disclosed in the prior art, the main objective of the invention is to provide an improved electrotherapeutic apparatus which detects a vibrational frequency of heart blood vessel and then produces a stimulating current corresponding to the vibrational frequency of heart blood vessel and the method of controlling the same.

Another objective of the invention is to provide an improved electrotherapeutic apparatus which detect a vibrational frequency of heart blood vessel and then produce a stimulating current corresponding to the vibrational frequency of heart blood vessel and then enhance the therapeutic effect of pain and rehabilitation by adjusting stimulating signal frequency according to the stimulating current.

In order to achieve the aforementioned requirements, the invention provides an improved electrotherapeutic apparatus which detects a vibrational frequency of heart blood vessel and then produces a stimulating frequency current corresponding to the vibrational frequency of heart blood vessel. The improved electrotherapeutic apparatus comprises: a detecting module which detects a vibrational frequency of heart blood vessel and produces a signal of the vibrational frequency of heart blood vessel, a processing module which receives the signal of the vibrational frequency of heart blood vessel and produces a response signal, and a action module which receives the response signal and then produces a stimulating current corresponding to the vibrational frequency of heart blood vessel, and enhance the therapeutic effect of pain and rehabilitation by adjusting stimulating signal frequency according to the stimulating current.

### BREIF DESCRIPTION OF THE DRAWINGS

The accompanying drawings incorporated in and forming a part of the specification illustrate several aspects of the present invention, and together with the description serve to explain the principles of the disclosure. In the drawings:
FIG 1 is a drawing of the therapeutic apparatus 1 disclosed in TW M428769;
FIG 2 is a drawing of the improved electrotherapeutic apparatus 2 disclosed in the invention ;
FIG 3 is a drawing of the processing module 22 disclosed in the invention;
FIG 4 is a drawing of the action module 23 disclosed in the invention; and
FIG 5 is a process flow diagram of controlling the electrotherapeutic apparatus disclosed in the invention.

### DETAILED DESCRIPTION OF THE PREFERRED EMBODIMENTS

Accordingly, some embodiments will be provided in the following section. For a person have an ordinary skill in the art can fully understand the advantage and effect of the electrotherapeutic apparatus and controlling method disclosed in present invention.

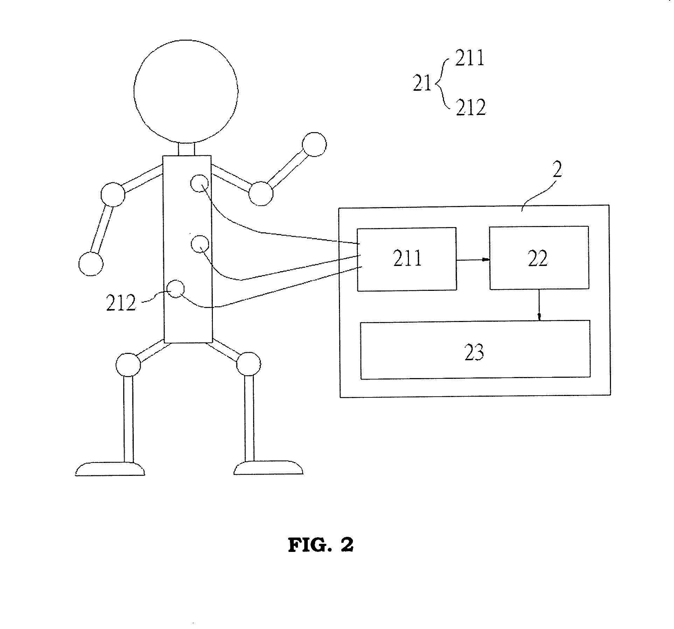

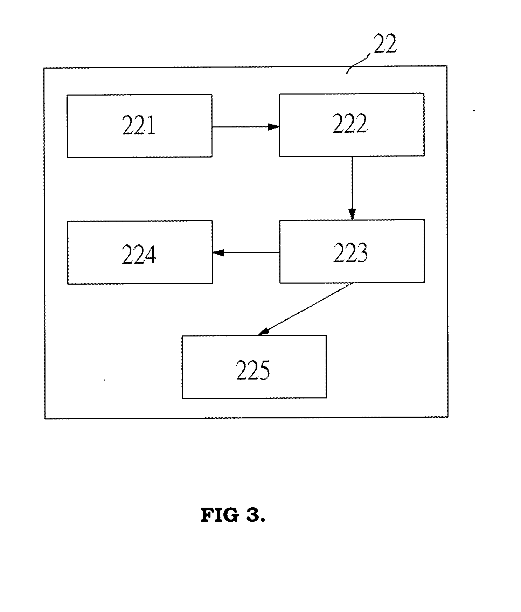

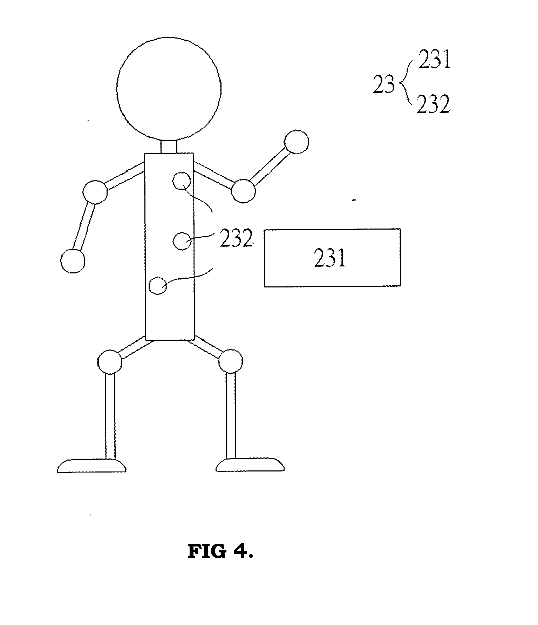

In a representative embodiment of the present invention, please refer to FIG 2, FIG 2 is a drawing of the improved electrotherapeutic apparatus 2 which detects a vibrational frequency of heart blood vessel and then produces a stimulating frequency current corresponding to the vibrational frequency of heart blood vessel. The improved electrotherapeutic apparatus 2 comprises: a detecting module 21 which detects a vibrational frequency of heart blood vessel and produces a signal of the vibrational frequency of heart blood vessel, a processing module 22 which receives the signal of vibrational frequency of heart blood vessel and produces a response signal, and a action module 23 which receives the response signal and then produces a stimulating current corresponding to the vibrational frequency of heart blood vessel. The detecting module 21 further comprises an electrocardiography 211 and plural testing units 212. The plural testing units 212 are pasted onto the body and obtain vibration frequency of heart blood vessel by adjusting the stimulating frequency to 0~3.3V. Referring to FIG 3, FIG 3 is a drawing of the processing module 22 which comprises a low pass filtering unit 221, an analog-to-digital converter unit 222, a cardiac electrical triggering calculating unit 223, a signal isolating unit 224 and a waveform adjusting unit 225. As the detecting module 21 measures the vibrational frequency of heart blood vessel, the signal of the vibrational frequency of heart blood vessel is transferred to the processing module 22. The low pass filtering unit 221 in the processing module 22 reduces the noise of the signal of the vibrational frequency of heart blood vessel and the analog-to-digital converter unit 222 converts the analog signal to the digital signal. The digital signal is further transferred to the cardiac electrical triggering calculating unit 223 and produces a cardiac electrical triggering signal. The cardiac electrical triggering signal is transferred to the signal isolating unit 224 and then to the waveform adjusting unit 225 to produce a response signal with a voltage controlling device. Referring to FIG 4, FIG 4 is a drawing of the action module 23 which comprises an electro-stimulating unit 231 and plural electrodes 232. The action module 23 receives the response signal from the waveform adjusting unit 225 and then produces a stimulating current corresponding to the vibrational frequency of heart blood vessel from the electro-stimulating unit 231. Therefore, the improved electrotherapeutic apparatus 2 enhances the therapeutic effect of pain and rehabilitation by adjusting stimulating signal frequency according to the stimulating current.

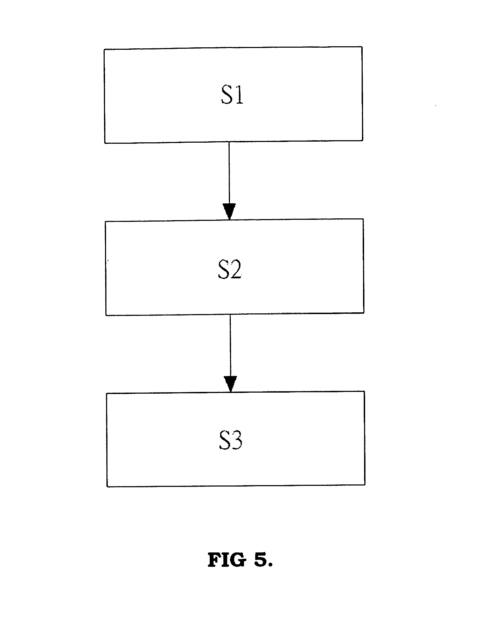

Refer to FIG 5, FIG 5 is a process flow diagram of controlling the electrotherapeutic apparatus disclosed in the invention. The steps are described as following:
Provides a detecting module which detects a vibrational frequency of heart blood vessel and produces a signal of the vibrational frequency of heart blood vessel S1.

Provides a processing module which receives the signal of vibrational frequency of heart blood vessel and produces a response signal S2.

Provides an action module which receives the response signal and then produces a stimulating current S3 corresponding to the vibrational frequency of heart blood vessel, and enhance the therapeutic effect of pain and rehabilitation by adjusting stimulating signal frequency according to the stimulating current S3.

Although specific embodiments have been illustrated and described, it will be obvious to those skilled in the art that various modifications may be made without departing from what is intended to be limited solely by the appended claims.

## Claims

1. An improved electrotherapeutic apparatus which detects a vibrational frequency of heart blood vessel and then produces a stimulating frequency current corresponding to the vibrational frequency of heart blood vessel, said improved electrotherapeutic apparatus comprising: a detecting module which detects a vibrational frequency of heart blood vessel and produces a signal of the vibrational frequency of heart blood vessel, a processing module which receives the signal of vibrational frequency of heart blood vessel and produces a response signal ; and an action module which receives the response signal and then produces a stimulating current corresponding to the vibrational frequency of heart blood vessel, and enhance the therapeutic effect of pain and rehabilitation by adjusting stimulating signal frequency according to the stimulating current.

2. The improved electrotherapeutic apparatus of claim 1, wherein the detecting module comprises: an electrocardiography and plural testing units.

3. The improved electrotherapeutic apparatus of claim 2, wherein the plural testing units are pasted onto all parts of body.

4. The improved electrotherapeutic apparatus of claim 1, wherein the processing module comprises: a low pass filtering unit, a analog-to-digital converter unit, a cardiac electrical triggering calculating unit, a signal isolating unit and a waveform adjusting unit.

5. The improved electrotherapeutic apparatus of claim 1, wherein the action module comprises: an electro-stimulating unit and plural electrodes.

6. A method of controlling electrotherapeutic apparatus which detects a vibrational frequency of heart blood vessel and then produces a stimulating current corresponding to the vibrational frequency of heart blood vessel, said method comprising :
providing a detecting module which detects a vibrational frequency of heart blood vessel and produces a signal of the vibrational frequency of heart blood vessel,
providing a processing module which receives the signal of vibrational frequency of heart blood vessel and produces a response signal; and
providing a action module which receives the response signal and then produces a stimulating current corresponding to the vibrational frequency of heart blood vessel, and enhance the therapeutic effect of pain and rehabilitation by adjusting stimulating signal frequency according to the stimulating current.

7. The method of claim 6, wherein the detecting module is an electrocardiography.

8. The method of claim 7, wherein the plural testing units are pasted on body and arms.

9. The method of claim 6, wherein the processing module comprises: a low pass filtering unit, a analog-to-digital converter unit, a cardiac electrical triggering calculating unit, a signal isolating unit and a waveform adjusting unit.

10. The method of claim 6, wherein the action module comprises: an electro-stimulating unit and plural electrodes.
